# EUROPEAN PATENT APPLICATION

(11) **EP 3 878 941 A1**
(43) Date of publication of application: **15.09.2021**
(21) Application number: 19883197.6
(22) Date of filing: 07.11.2019
(51) Int. Cl.: C12M 3/00, C12N 5/02, C12N 5/071, C12N 5/077, C12N 5/079

(54) **ANIMAL CELL GROWTH PROMOTER, CULTURE MEDIUM FOR ANIMAL CELL CULTURE, AND ANIMAL CELL CULTURE APPARATUS**

(30) Priority: 08.11.2018 JP 2018210910
(71) Applicant: IntegriCulture Inc., Tokyo 113-0033 (JP)
(72) Inventor: KAWASHIMA, Ikko, Tokyo 113-0033 (JP); HANYU, Yuki, Tokyo 113-0033 (JP); FUKUMOTO, Keita, Tokyo 113-0033 (JP)
(74) Representative: Grünecker Patent- und Rechtsanwälte PartG mbB
(86) International application number: PCT/JP2019/043713
(87) International publication number: WO 2020/096004

(57) **Abstract**

The present invention aim at providing a novel animal cell growth promoter. Specifically, a culture supernatant of an embryonic membrane derived from an avian or reptilian fertilized egg is use as an animal cell growth promoter. For example, by adding, to an animal cell culture medium, an animal cell growth promoter comprising, as an active ingredient, a culture supernatant of an embryonic membrane derived from an avian or reptilian fertilized egg, the animal cell growth can be promoted.

## Description

### Cross reference to related application

This application claims priority to Japanese Patent Application No. 2018-210910, filed November 8, 2018, which is incorporated herein by reference.

### Technical field

The present invention relates to animal cell growth promoters, media for culturing animal cells, and devices for culturing animal cells.

### Background art

In the culture of animal cells, sera-containing culture media supplemented with a fetal bovine serum or with hormones or growth factors in place of the sera as a factor with activity for maintaining cell growth.

Besides those factors, JP-A-2013-247927 discloses that rice bran extracts have growth-promoting effects on cultured animal cells. JP-A-2011-182736 discloses that media supplemented with hydrolyzed β-conglycinin concentrate enhance cell growth rate when animal cells are cultured in the media. Furthermore, JP-A-7-188292 discloses that glycoproteins in the supernatant from cultures of human-derived fibroblasts promote human vascular endothelial and liver parenchymal cell growth. In addition, JP-A-5-301893 discloses that a protein in the conditioned madia obtained by culturing human-derived glioma cell lines has a growth-promoting effect on glial cells and fibroblasts.

### Summary of the invention

### Problem to be solved by the invention

An object of the present invention is to provide novel animal cell growth promoters, media for culturing animal cells, and devices for culturing animal cells.

### Means for solving the problem

An aspect of the present invention is an animal cell growth promoter including, as an active ingredient, a culture supernatant of an embryonic membrane from an avian or reptilian egg. The egg may comprise a chicken egg. The animal cell growth promoter may be the one for a muscle cell, a visceral cell, or a neural cell. The animal cell growth promoter may be the one for a liver cell, a pancreas cell, or an oviduct cell.

Another aspect of the present invention is a cell culture medium containing any one of the cell growth promoters described above.

Another aspect of the present invention is a device for culturing animal cells including: a first culture vessel for culturing cells derived from an embryonic membrane of an avian or reptile egg; a second culture vessel for culturing animal cells to be propagated; a first flow path for flowing a culture medium from the first culture vessel to the second culture vessel; a second flow path for flowing a culture medium from the second culture vessel to the first culture vessel; and a medium-flow-rate control unit for circulating a culture medium in the order of the first culture vessel, the first flow path, the second culture vessel, and the second flow path, and controlling the flow of the culture medium in the first flow path and the second flow path depending on a condition of the animal cells and/or a condition of the culture medium. The device may include a culture medium introduction path for introducing a fresh culture medium to the circulating culture medium; and a culture medium discharge path for discharging a used culture medium, from the circulating culture medium, and the medium-flow-rate control unit may controls the introduction of a fresh culture medium via the culture medium introduction path and the discharge of the used culture medium, via the culture medium discharge path, depending on a condition of the animal cells and/or a condition of the culture medium.

### Brief description of the drawings

Fig. 1 is a schematic diagram of a device for growing cells according to an embodiment of the present invention;
Fig. 2 shows photographs of observations showing that growth of the cells derived from the tissues shown in the figure (stomach, skeletal muscle, heart, gallbladder, intestine, brain, bursa of Fabricius, and bone) is promoted when a culture supernatant of chicken egg chorioallantoic membranes is added to the medium in an embodiment of the present invention;
Fig. 3 shows graphs quantitatively representing the results in Fig. 1 for cells derived from the liver, pancreas, and oviduct in an embodiment of the present invention; and
Fig. 4 shows graphs showing that growth of the cells derived from the tissues shown in the figure (stomach, muscle, heart, liver, intestine, brain, bursa of Fabricius, and bone)is promoted when a culture supernatant of chicken egg yolk sacs is added to the medium in an embodiment of the present invention.

### Detailed description of the invention

Objects, characteristics, advantages, and ideas of the present invention are apparent to a person skilled in the art from the description of the present specification and the person skilled in the art can easily reproduce the present invention from the description of the present specification. Embodiments of the present invention, specific examples thereof and so forth, which are described in the following, indicate preferable embodiments of the present invention and are described for exemplification or explanation, and the present invention is not limited thereto. It is apparent to the person skilled in the art that various alterations and modifications can be made on the basis of the description of the present specification within the spirit and the scope of the present invention disclosed in the present specification.

### == Animal cell growth promoter ==

Animal cell growth promoters of the present disclosure contain, as an active ingredient, a culture supernatant of an embryonic membrane from an avian or reptilian egg.

Examples of the birds and reptiles from which eggs as a source for embryonic membranes are collected may include, but are not limited to, quail, chickens, lizards, snakes, crocodilians, and turtles. The birds and reptiles may be of any species having embryonic membranes in their eggs.

As used herein, the term "embryonic membrane" refers to a membrane tissue that is formed outside the embryonic body, performs functions such as protection of the embryo, nutrition provision and respiration, and does not participate in the formation of the body after hatching or birth, in developmental processes of birds and reptiles. Examples include the chorion that is the outermost part around an embryo, the amnion surrounding the embryo body, the allantois that forms the urinary bladder, the chorioallantoic membrane (CAM) formed by partial fusion of the chorion and the allantois, and the yolk sac enclosing the yolk.

The term "culture supernatant of an embryonic membrane" refers to the medium in which an isolated embryonic membrane has been cultured. The embryonic membrane to be cultured may be alone or a combination of membranes such as the chorion, amnion, allantois, allantochorion, and yolk sac as described above.

Embryonic membranes can be obtained from eggs using a known method (see, for example, Eiji Ichishima, "KAGAKU TO SEIBUTSU", vol. 13, issue 8, p. 489-497 (1975)). Specifically, for example, embryonic membranes can be isolated by the following method. First, a fertilized egg is incubated under conditions suitable for artificial incubation for a certain period of time, and then the eggshell is cracked. (1) The allantois can be obtained by picking up the swollen membrane generated from the embryo's ventral side of the posterior gastrointestinal tract. (2) The amnion can be obtained by picking up the transparent membrane tissue with no blood vessels around the embryo. (3) The yolk sac can be obtained by picking up the membrane tissue enclosing the yolk. (4) The chorion can be obtained by picking up the outermost membrane tissue surrounding all the elements in the egg. Furthermore, as the incubation time is increased, the chorion and the allantois partially fuse to form the chorioallantoic membrane. At this stage, the chorioallantoic membrane can be obtained by picking up the membrane tissue that is in contact with the eggshell and has blood vessels running through it. The incubation period can appropriately be determined depending on the kind of animal as well as the membrane tissue of interest. For example, for chicken eggs, 4-21 days are preferable, 10-18 days are more preferable, and 13-15 days are even more preferable. When culturing these embryonic membranes, they may be cultured in their membranous form or as embryonic membranes-derived cells isolated from the embryonic membrane by physically breaking the membrane or by enzymatically treating the membrane with proteases (e.g., collagenase, elastase, dispase, papain, etc.).

For culturing the embryonic membranes, media used for typical animal cell culture can be used, and examples include, but are not limited to, DMEM and F12. To the media, supplements that are usually formulated as medium additives may be added. The culture conditions can also be those generally used for animal cell cultures. Typically, cells are cultured at 5% CO₂ and 37°C in a carbonate buffer-based medium. However, the culture conditions are not limited to the one mentioned above and can appropriately be selected by those skilled in the art. The incubation time is also not limited, but 1-7 days are preferable, and 2-6 days are more preferable. After culturing, the supernatant may be collected and added directly to the culture media or the supernatant from which solids such as cell residues have been removed by filtration or centrifugation, may be added. The amount of the culture supernatant added to the culture media is not particularly limited as long as it is an effective amount by which the effect for cell growth can be observed. Still, the final concentration in the media is preferably 20% or more, more preferably 35% or more, and even more preferably 50% or more.

Any type of animal cells can be cultured in the culture media to which this culture supernatant is added, and examples include muscle cells, such as smooth muscle, cardiac muscle, and skeletal muscle cells; visceral cells, such as cardiac, liver, stomach-derived, and intestine-derived cells; and neural cells, such as neurons and glial cells. Any species of animals can be used as a source of the cells and examples include human, murine, rats, primate, and swine.

Thus, by adding the culture supernatant obtained by culturing cells derived from an embryonic membrane to the animal cell culture media, the growth of the animal cells in culture can be promoted. Therefore, this culture supernatant can be used as an animal cell growth promoter.

When the culture supernatant of cells derived from the embryonic membrane is used as an animal cell growth promoter, other cell growth factors may be used simultaneously.

### == Device for growing cells ==

A device for growing cells according to the present invention includes a first culture vessel for culturing cells derived from an embryonic membrane of an avian or reptile egg; a second culture vessel for culturing animal cells to be propagated; a first flow path for flowing a culture medium from the first culture vessel to the second culture vessel; a second flow path for flowing a culture medium from the second culture vessel to the first culture vessel; and a medium-flow-rate control unit for circulating a culture medium in the order of the first culture vessel, the first flow path, the second culture vessel, and the second flow path, and controlling the flow of the culture medium in the first flow path and the second flow path depending on a condition of the animal cells and/or a condition of the culture medium.

In the first culture vessel, cells are cultured according to the method of culturing cells derived from an embryonic membrane, which is performed in preparing animal cell growth promoters, as described above. In the second culture vessel, cells are cultured according to the method of culturing animal cells in a medium to which an animal cell growth promoter is added, as described above.

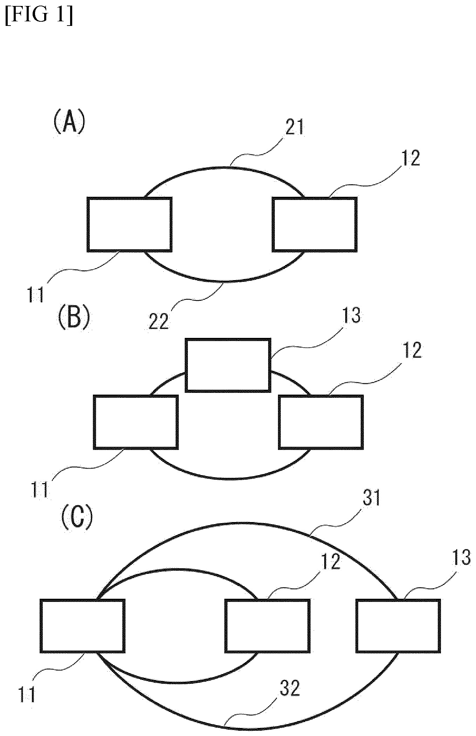

The first and second culture vessels are fluid-connected to each other via the first and second flow paths. For example, as shown in Fig. 1(A), a first culture vessel 11 and a second culture vessel 12 may be directly connected to each other via a first flow path 21 and a second flow path 22. As long as the first culture vessel 11 and the second culture vessel 12 are fluid-connected to each other, one or more other culture vessels may be provided, and various configurations may be contemplated for the connections between the other culture vessel(s) and the first culture vessel 11 or the second culture vessel 12. For example, as shown in Figure 1(B), a third culture vessel 13 may be provided in the middle of the first flow path 21, and the flow path may be connected from the first culture vessel 11 to the second culture vessel 12 through the third culture vessel 13. For another example, as shown in Fig. 1(C), the third culture vessel 13 may be provided independently of the second culture vessel 12, and a third flow path 31 and a fourth flow path 32 fluid-connecting the first culture vessel 11 and the third culture vessel 13 may be provided separately from the first flow path 21 and the second flow path 22. In the case of Figs. 1(B) and 1(C), by appropriately selecting the cells to be cultured in the third culture vessel 13, the cells can be affected by the culture in the first culture vessel 11 or the culture in the second culture vessel 12, or can affect those cultures.

Here, the culture in the first culture vessel 11 and/or the culture in a culture vessel other than the second culture vessel 12 may get or impose various effects from or on the culture in the first culture vessel and/or the culture in the second culture vessel, and examples of such effects include cell growth, cell differentiation, tissue morphogenesis, pH control of the culture medium, and prevention of bacterial contamination.

For example, specifically, by placing cells in the third culture vessel 13 which are different from those in the second culture vessel 12 and can grow in a medium in which the cells derived from embryonic membrane were cultured, the cells in the second culture vessel 12 can be propagated simultaneously with those in the third culture vessel 13. For another example, by culturing, in the third culture vessel 13, cells derived from an embryonic membrane different from the one from which cells in the first culture vessel 11 have been obtained, the growth of cells in the second culture vessel 12 can be further promoted. For another example, the cells to be cultured in the third culture vessel 13 may be those which can grow in the medium in which cells derived from an embryonic membrane have been cultured and which secrete a factor promoting the growth of the cells in the second culture vessel 12.

The vessels may be connected to each other by tubes and the tubes may form the flow paths. These tubes may have valves installed therein. The medium-flow-rate control unit may monitor the conditions of the cultured cells and/or the medium for culturing cells and control the flow (e.g., a flow velocity, a flow rate, etc.) of the culture medium in each flow path via the valves depending on the conditions of the cultured cells and/or the medium for culturing cells.

The structure of each valve is not limited but may be any known type with, for example, a flange or a screw. Control mechanism of the values is not limited but may be any known mechanism of, for example, air-driven, electromagnetic, electric, and hydraulic type. The tubes may also be equipped with a check valve to prevent backflow of the culture medium in the tubes.

The condition of the cells and/or the condition of the medium for culturing cells monitored by the medium-flow-rate control unit include(s), but is/are not limited to, the progress of cell growth, the progress of cell differentiation, and the pH of the medium. The progress of cell growth can be determined by, for example, counting the number of cells per unit area or by measuring the ratio of the area occupied by cells to the bottom area of a plate. The progress of cell differentiation can be determined by, for example, introducing, into cells, a marker gene that emits fluorescence upon expression as the cells differentiate and measuring the fluorescence. The pH of each medium can be determined by, for example, measuring its pH directly or by adding a dye that changes when pH changes to the medium and judging the color of the dye.

### Examples

### (1) Isolation of chorioallantoic membranes and yolk sacs

Each of the chorioallantoic membranes and yolk sacs subjected to the tests were isolated as follows.

Fertilized chicken eggs were incubated at 37°C. After 14 days, the eggshells were cracked, and the membrane tissue in contact with the eggshell and having blood vessels running through it were isolated as the chorioallantoic membrane, and the membrane tissue enclosing the yolk were isolated as and the yolk sac. Each of the membrane tissues was immersed in a Hank's balanced salt solution (HBSS). The membrane tissue was physically cut off into small pieces using a tweezer or the like, and then was subjected to centrifugation and the supernatant was discarded.

A collagenase solution (200 IU/mL) was added to each isolated membrane tissue, which was then kept at 37°C for 1 hour and centrifuged again. The supernatant was discarded and then HBSS was added to the collagenase-treated membrane tissue, which was filtered through a 40-µm filter. The filtrate thus obtained was centrifuged and the supernatant was discarded. In this way, the cells derived from the chorioallantoic membrane and those derived from the yolk sac were separately collected. When the cells were stored, they were suspended in a cell-freezing solution (10% DMSO solution), frozen by gradual cooling to -80°C, and stored at -80°C.

### (2) Culture of chorioallantoic membranes and yolk sacs

The cells derived from the chorioallantoic membranes or the yolk sacs obtained in (1) were added to DMEM containing 10% fetal bovine serum (FBS) and a 1% penicillin-streptomycin (PS) solution (hereinafter FBS/PS-containing DMEM); seeded into cell culture dishes (10-cm diameter) at a concentration of 4 × 10⁶ cells/dish; and incubated for 2-4 days at 37°C in 5% CO₂.

### (3) Collection of supernatant

The culture media of the chorioallantoic membrane and yolk sac prepared by the culture method described in (2) were filtered through a 0.22-µm filter. The filtrates thus obtained were used as an animal cell growth promoter for animal cell culture. When the prepared supernatant was stored, it was frozen and stored at -20°C.

### (4) Animal cell culture

### (4-1) Isolation of cells from each tissue

Chick cells used for culture were isolated as follows.

First, fertilized chicken eggs were incubated at 37°C. After 14 days, the eggshells were cracked, and the chick embryos were picked up. Stomach, skeletal muscle, heart, gallbladder, intestine, brain, bursa of Fabricius, and bone were isolated from the chick 14-day embryos and each of them was immersed in a HBSS solution. Then, the cells derived from each tissue were isolated in the same way as in (1).

### (4-2) Culture of cells from each tissue

Cells derived from each tissue were cultured in the same way as (2) using a culture medium, i.e., the FBS/PS-containing DMEM supplemented with an equal amount of the culture supernatant of the chorioallantoic membrane or yolk sac prepared in (3). As a control, cells cultured under the same conditions except that the FBS/PS-containing DMEM without the culture supernatant of the chorioallantoic membrane or yolk sac was used.

### (4-3) Counting the number of cells in each culture

The results of (4-2) were quantitatively evaluated for cells derived from the liver, pancreas, oviduct, stomach, muscle, heart, intestine, brain, bursa of Fabricius, and bone. Here, the number of cells derived from each tissue was counted as follows.

The culture medium of the cells derived from each tissue was discarded from the dishes, and PBS was added to wash the cells. After PBS was discarded, a trypsin-EDTA solution was added. The cells were incubated at 37°C for 1-2 minutes to be detached from the dishes, and the FBS/PS-containing DMEM was added to stop the reaction. All the solutions containing the detached cells were collected and centrifuged to obtain the cells. To these cells, FBS/PS was added to suspend the cells, and 20 µL of this cell suspension was mixed with 20 µL of trypan blue solution. The number of live cells in this cell suspension, which were not stained with trypan blue, was counted under a microscope using a blood cell calculator.

### (4-4) Results

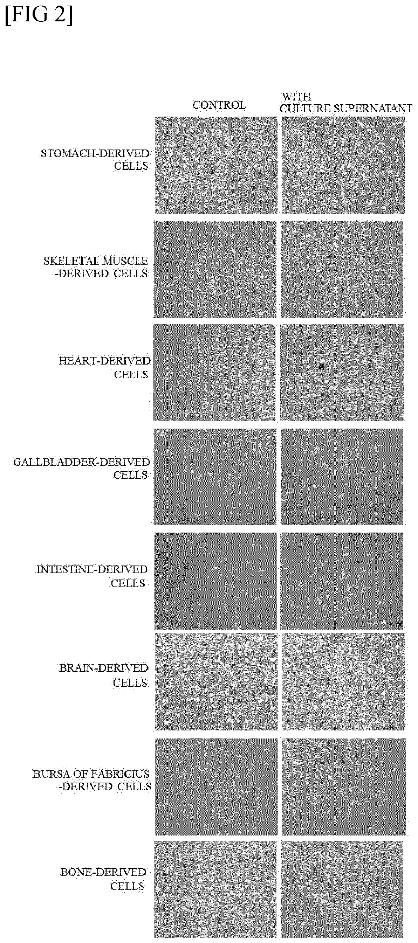

First, the cells were observed under a phase-contrast microscope when they were cultured for approximately 3 - 5 days in a medium supplemented with the culture supernatant of the chorioallantoic membrane. Fig. 2 shows photographs of the cells, in which more cells were observed when the culture supernatant prepared in (3) was added than when the culture supernatant was not added. This indicates that the added culture supernatant promoted the growth of the cells.

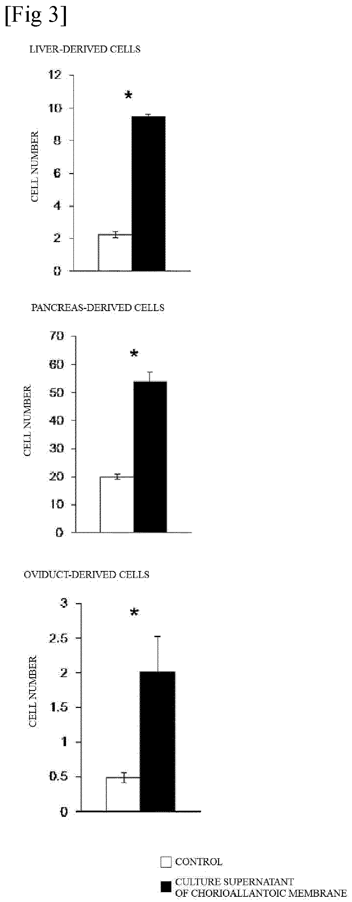

To quantitatively demonstrate this result, the mean and standard errors for the number of cells derived from the liver, pancreas, and oviduct in the respective three Petri dishes using the culture supernatant of the chorioallantoic membrane are shown in Fig. 3. For all cases of the cells derived from the liver, pancreas, and oviduct, the addition of the culture supernatant resulted in an approximately 2.5- to 4-fold increase in cell growth compared with the cells grown without such supernatant.

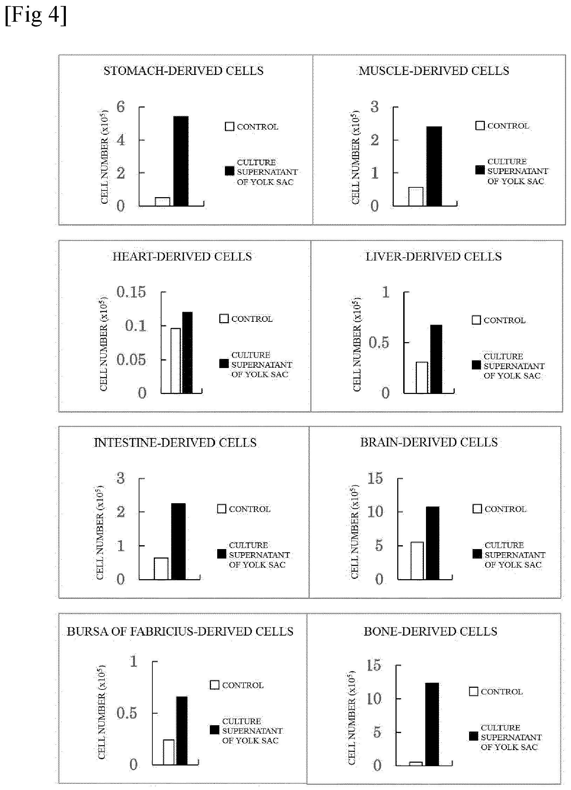

Next, the growth-promoting effect of the culture supernatant of the yolk sac on the cells derived from the stomach, muscle, heart, liver, intestine, brain, bursa of Fabricius, and bone was investigated using media containing the culture supernatant in the same way (n = 2). As shown in Fig. 4, in all cases, more cells were observed when the culture supernatant of the yolk sac was added than when the culture supernatant was not added. This indicates that the addition of the culture supernatant of the yolk sac promoted the cell growth.

### Industrial applicability

The present invention has made it possible to provide novel animal cell growth promoters, media for culturing animal cells, and devices for culturing animal cells.

## Claims

1. An animal cell growth promoter comprising, as an active ingredient, a culture supernatant of an embryonic membrane derived from an avian or reptilian fertilized egg.

2. The animal cell growth promoter according to Claim 1, wherein the embryonic membrane is at least one selected from an amnion, a chorioallantoic membrane, and a yolk sac.

3. The animal cell growth promoter according to Claim 1 or 2, wherein the egg comprises a chicken egg.

4. The animal cell growth promoter according to any one of Claims 1 to 3 for a muscle cell, a visceral cell, or a neural cell.

5. The animal cell growth promoter according to any one of Claims 1 to 3 for a liver cell, a pancreas cell, or an oviduct cell.

6. A cell culture medium comprising the animal cell growth promoter according to any one of Claims 1 to 5.

7. A device for culturing animal cells comprising:
a first culture vessel for culturing cells derived from an embryonic membrane of an avian or reptile egg;
a second culture vessel for culturing animal cells to be propagated;
a first flow path for flowing a culture medium from the first culture vessel to the second culture vessel;
a second flow path for flowing a culture medium from the second culture vessel to the first culture vessel; and
a medium-flow-rate control unit for circulating a culture medium in the order of the first culture vessel, the first flow path, the second culture vessel, and the second flow path, and controlling the flow of the culture medium in the first flow path and the second flow path depending on a condition of the animal cells and/or a condition of the culture medium.

8. The device according to Claim 7, further comprising:
a culture medium introduction path for introducing a fresh culture medium to the circulating culture medium and
a culture medium discharge path for discharging the used culture medium from the circulating culture medium, and wherein
the medium-flow-rate control unit controls the introduction of the fresh culture medium via the culture medium introduction path and the discharge of the used culture medium, via the culture medium discharge path depending on a condition of the animal cells and/or a condition of the culture medium.
